# EUROPÄISCHE PATENTSCHRIFT

(11) **EP 3 099 359 B1**
(45) Veröffentlichungstag und Bekanntmachung des Hinweises auf die Patenterteilung: **20.03.2019**
(21) Anmeldenummer: 15704454.6
(22) Anmeldetag: 20.01.2015
(51) Int. Cl.: A61M 11/00, A61M 15/00, A61M 15/08

(54) **PULVERINHALATOR UND PULVERINHALATIONSSET**
POWDER INHALER AND POWDER INHALATION SET
INHALATEUR DE POUDRE ET SET POUR L'INHALATION DE POUDRE

(30) Priorität: 30.01.2014 DE 102014001072; 17.04.2014 DE 102014005646
(43) Veröffentlichungstag der Anmeldung: 07.12.2016
(73) Patentinhaber: Beller, Klaus-Dieter, 79341 Kenzingen (DE)
(72) Erfinder: Beller, Klaus-Dieter, 79341 Kenzingen (DE)
(74) Vertreter: Mehl-Mikus, Claudia
(86) Internationale Anmeldenummer: PCT/EP2015/000089
(87) Internationale Veröffentlichungsnummer: WO 2015/113742

(56) Entgegenhaltungen:
- EP-B1- 2 015 812
- WO-A1-93/18811
- WO-A1-2011/154371
- WO-A2-97/25086
- WO-A2-2013/036881
- DE-A1- 4 400 084
- DE-A1-102009 041 664
- GB-A- 2 270 293
- GB-A- 2 460 281

## Beschreibung

Die Erfindung betrifft einen Pulverinhalator und ein Pulverinhalationsset aus Pulverinhalator und Blisterelement.

Pulverinhalatoren zur sublingualen, nasalen und inhalativen Applikation pulverförmiger, feststofflicher Arzneimittel oder anderer Mitteln sind bekannt. Sie können als treibgasfreie Applikationsvorrichtungen ausgestaltet sein und setzen ein Aerosol durch den Inspirationsvorgang, respektive einen tiefen Inhalationsvorgang frei. Die Energie für die Dispergierung wird dabei durch den inspiratorischen Fluss gewonnen. Die pulverförmige Substanz ist dabei in einem Vorratsbehältnis enthalten; es sind auch spezielle Vorratsbehältnisse wie Blister bekannt. Je nach Art des Pulverinhalators wird der reine Wirkstoff oder es wird der Wirkstoff mit einem Träger, der ein unbedenklicher Hilfsstoff wie bspw. Lactose oder Glucose für adhährierte Wirkstoffpartikel ist, eingesetzt.

Bekannte Pulverapplikatoren bzw. Pulverinhalatoren können je nach Ausgestaltung bis zu drei verschiedene Sorten von Pulvern verabreichen. Einen solchen Pulverinhalator beschreibt die EP 1769 818 B1: Dort wird ein treibgasfreier Pulverinhalator zur inhalativen Applikation pulverförmiger feststofflicher Arzneimittel eingesetzt, der es auch erlaubt, eine Pulverkombination zu inhalieren. Dies wird dadurch erreicht, dass der Pulverinhalator wenigstens zwei Vorratsbehältnisse aufweist, in denen unterschiedliche Pulver voneinander getrennt in mehreren Dosiereinheiten bevorratet sind. Die Pulver können entsprechend zusammengeführt und über ein gemeinsames Inhalationsrohr verabreicht werden.

Ebenfalls für die Inhalation zweier unterschiedlicher Pulver vorgesehen ist der Pulverinhalator aus DE 10 2005 046 645 B3, der für die unterschiedlichen Pulver ebenfalls wenigstens zwei Vorratsbehältnisse aufweist und es ermöglicht, dass diese getrennt voneinander in einer Dosiereinrichtung dosiert und anschließend während des Inhalierens miteinander vermischt werden.

Die Verabreichung auch unterschiedlicher Pulver kann als eine Einzeldosis erfolgen.

Um den Agglomerationsgrad und den Entleerungsgrad des Pulvers beim Inhalieren zu verbessern, weist der Pulverinhalator der DE 10 2005 046 644 B3 im Zuluftkanal eine wendel- oder spiralförmige Verwirbelungseinrichtung auf, um die Luft vor der Zufuhr zu dem Pulver zu verwirbeln und so eine feinere Verteilung des Pulvers im Luftstrom zu erreichen.

Ein Inhalator für Einzeldosen ist aus der DE 20 2011 103 503 U1 bekannt. Der dort beschriebene Pulverinhalator für Kapseln umfasst ein Klingengehäuse und ein Mundstückgehäuse, sowie einen Kapselträger und mehrere Klingen. Der Pulverinhalator dort soll ohne komplizierte Vorbereitungen durch einen ungeübten Benutzer eingesetzt werden können. Der gesamte Inhalationsvorgang soll durch lediglich eine Schiebebewegung aktiviert werden können, dazu weist die Vorrichtung dort nur zwei zueinander bewegliche Gehäuseteile auf, durch deren Verschiebung zueinander zunächst die Einlegeöffnung für die Kapsel verschlossen und dadurch die Kapsel in einer Bohrung im Inhalator zurück gehalten wird. Im Inneren des Inhalators wird die Kapsel dann mit geeignet geformten, metallischen Klingen geöffnet.

Aus EP 2 015 812 B1 ist ein Pulverinhalator zur gleichzeitigen Verabreichung von mehreren Medikamenten bekannt, die in einem Einzeldosis-Blisterstreifen in verschiedenen Mulden vorliegen. Zum Einlegen des Blisters ist ein Mundstück des Pulverinhalators beweglich an einer Streifenauflage angesetzt. An der Streifenauflage des Pulverinhalators sind entsprechende Mulden zur Aufnahme der Blistermulden ausgeformt. Eine Deckfolie des eingelegten Blisters lässt sich bei geschlossenem Mundstück abziehen, indem die Deckfolie einen aus dem geschlossenen Pulverinhalator herausragenden umgeschlagenen Überlapp aufweist.

Während die meisten bekannten Inhalatoren aus einer Vielzahl von Bauteilen bestehen, was die Fertigung sehr teuer und aufwändig gestaltet, ist der oben genannte Inhalator DE 20 2011 103503 U1 bereits konstruktiv einfach und günstig fertigbar gestaltet; verlangt aber den Einsatz von Klingen und verfügt nur über einen kurzen, einfachen Transportweg um das Pulver aus der Kapsel mittels eingezogener Luft in ein entsprechendes Aerosol zu überführen.

DE 44 00 084 A1 beschreibt einen Pulverinhalator aus einer Folienabwicklung, die zwei flache, längliche und durch Wandungsabschnitte berandete Abschnitte mit Pulveraufnahmemulden aufweist. Durch Falten der Folienabwicklung und Verbinden der Kanten wird ein längliches Gehäuse mit einem Mundstück an einer Schmalseite und einem gegenüberliegenden Lufteinlass geformt. Im Inneren der zusammengefügten Folienabwicklung kann ein kreuzförmiger Steg den Luftkanal aufteilen, und zum Öffnen der Pulveraufnahmemulden kann ein Dorn in jeder Mulde vorgesehen sein.

In WO 2013/036881 A2 ist ein dreiteiliger Pulverinhalator beschrieben, bei dem der Pulver-Luftauslassbereich zum Mundstück hin ein separates Bauteil ist. In einem zweiten Bauteil ist eine Torsionskammer ausgeformt, in die Lufteinlasskanäle, die in einem dritten Bauteil ausgeformt sind, nach Zusammenfügen der Bauteile münden. Das dritte Bauteil weist außerdem eine Auslassgitteröffnung auf, die auf der Torsionskammer zu liegen kommt und an der das separate Auslassstück befestigt wird. Dieser Inhalator ist zur Einmalverwendung vorgesehen, die Bauteile werden miteinander verklebt, ein Öffnen des Pulverinhalators nach der Verwendung ist nicht vorgesehen.

WO 2011/154371 A1 offenbart einen Pulverinhalator mit sehr komplexem Aufbau, der aus einer Mehrzahl von Teilen besteht. Eine Vielzahl von Lufteinlassöffnungen ist in dem Deckel vorgesehen, der einen gebogenen Luftstromkanal schließt. Die Lufteinlassöffnungen erstrecken sich flächig über den Luftstromkanal, der einen Direktkanalabschnitt, der direkt zur Wirbelkammer führt, und einen Behälterkanalabschnitt aufweist, der die Aufnahmemulde passiert, in der ein Blister mit seiner Wirkstoffmulde aufgenommen ist. Eine vom Deckel in Richtung der Aufnahme weisende Rippe sorgt für eine Umlenkung des Luftstromkanals in die Wirkstoffmulde.

GB 2 270 293 A beschreibt einen Pulverinhalator aus zwei Halbschalen, von denen die obere Halbschale mit Membranen verschlossene Bohrungen aufweist, in denen jeweils eine Pulverdosis vorliegt. Die Membran wird durch Eindrücken einer Kappe mit Schneidkante geöffnet, sodass das Pulver in den Pulverfreigabebereich gelangt. Der Inhalator weist gewundene Zuluftkanäle auf, die sich von den Lufteinlassöffnungen zu dem Pulverfreigabebereich erstrecken und für Luftverwirbelung sorgen.

Der in GB 2460281 beschriebene Inhalator besteht aus mehreren aufeinander laminierten Schichten, sodass er die Größe einer Kreditkarte besitzt. Die mittlere Schicht weist Ausschnitte für den Lufteinlassbereich, eine verjüngende Luftpassage, die Medikamentenkammern und den Luftauslassbereich auf. In der Deckschicht sind ein Lufteinlass, der über dem Lufteinlassbereich der mittleren Schicht zu liegen kommt, und Entlüftungsöffnungen, die über jeder Medikamentenkammer zu liegen kommen. Zwischen der mittleren und der Bodenschicht kann eine weitere Schicht mit einer Ausnehmung unter der Luftpassage in der mittleren Schicht vorgesehen sein. Diese Ausnehmung, in der das Medikament vorliegt, wird durch einen umgeklappten Folienstreifen versiegelt, der durch den Lufteinlass herausragt und zum Öffnen der Ausnehmung abgezogen werden kann. Aus DE 10 2009 041 664 A1 ist eine Pulverinhalator für ein in einem Blister verpacktes Pulver bekannt. Der Pulverinhalator besteht aus zwei Gehäuseteilen, die in vorbestimmter gegenseitiger Lage in einen aktivierten Zustand zusammenfügbar sind. Das Gehäuse bildet dabei einen an einem Einströmende und an einem Ausströmende offenen Luftströmungskanal aus und weist eine Einrichtung zur Aufnahme der Blistermulde mit dem pharmazeutischen Pulver sowie eine Vorrichtung zum Öffnen des Blisters auf, die beim Zusammenfügen der Gehäuseteile aktiviert wird. Das eine Gehäuseteil, in dem die Einrichtung zur Aufnahme der Blistermulde ausgebildet ist, ist ein längliches, flaches Unterteil. Das zweite Gehäuseteil mit der Vorrichtung zum Öffnen der Blistermulde ist ein längliches, flaches Oberteil, wobei die Vorrichtung zum Öffnen der Blistermulde als ein Vorsprung ausgebildet ist, mit dem die Deckfolie des Blisters beim Zusammenfügen der Gehäusteile durchstoßen wird. Wenigstens ein Gehäuseteil weist an seinen Längsrändern vorstehende Leisten auf, die im zusammengefügten Zustand der Gehäuseteile im Zusammenwirken mit dem anderen Gehäuseteil den Luftströmungskanal an seinen Längsseiten begrenzen.

Ausgehend von diesem Stand der Technik ist es Aufgabe der vorliegenden Erfindung, einen noch einfacher fertigbaren Inhalator zu schaffen, der auch bei mehrfacher Verwendung hygienisch einwandfrei bleibt.

Diese Aufgabe wird mit dem Pulverinhalator mit den Merkmalen des Anspruchs 1 gelöst. Entsprechend ergibt sich weiter die Aufgabe, ein Pulverinhalationsset zu schaffen, das den Inhalator samt Wirkstoff direkt einsetzbar bereitstellt.

Diese Aufgabe wird durch das Pulverinhalationsset mit den Merkmalen des Anspruchs 21 gelöst.

Weiterbildungen der Vorrichtung und des Verfahrens sind in den Unteransprüchen ausgeführt.

Der erfindungsgemäße Pulverinhalator umfasst zwei Halbschalen, die durch ein Filmscharnier miteinander verbunden und einstückig ausgebildet sind. Die beiden Halbschalen umgeben in einer zusammengefügten Anordnung einen Lufteinlassbereich, einen Pulverdepositions- und -freigabebereich und einen Auslassbereich, wobei durch all diese Bereiche ein Fluidpfad verläuft. Dabei hat eine der beiden Halbschalen in dem Pulverdepositions- und -freigabebereich wenigstens eine Pulveraufnahmemulde, in die der durch den Lufteinlassbereich zugeführte Luftstrom geleitet wird. In der Pulveraufnahmemulde kann eine Mulde eines zur Aufnahme in dem Pulverinhalator dimensionierten Blisterelements aufgenommen werden, das eine Trägerfolie aufweist, die zumindest die Mulde aufweist, die zur Aufnahme zumindest eines inhalierbaren Wirkstoffpulvers ausgebildet ist. Dabei verschließt eine Deckelplatte die Mulde, die in Größe und Position entsprechend zur Aufnahme in der zumindest einen Pulveraufnahmemulde des Pulverinhalators ausgebildet ist. Dabei wird das Pulver vollständig aus der Pulveraufnahmemulde ausgetragen und bereits zumindest teilweise desagglomeriert. Im anschließenden Auslassbereich ist eine oder sind mehrere Desagglomerationsstrukturen vorgesehen, um das Pulver weiter bzw. noch feiner zu desagglomerieren und dessen Absetzen zu verhindern. Wenigstens eine der beiden Halbschalen verfügt in dem Lufteinlassbereich über eine oder mehrere Lufteinlassöffnungen und beide Halbschalen zusammen bilden im Auslassbereich einen Auslass, resp. eine Auslassöffnung. Auf diese Weise bilden die beiden Halbschalen im zusammengefügten Zustand ein Strömungsgehäuse für den Fluidpfad zwischen den Lufteinlassöffnungen und dem Auslass, sodass das Aerosol, das beim Einatmen in dem Pulverdepositions- und - freigabebereich aus zu inhalierendem Pulver, das in der Pulveraufnahmemulde bereitgestellt wird, und Atemluft gebildet wird, durch den Auslass vom Anwender inhaliert werden kann. Es wird vorteilhaft lediglich ein einziges Bauteil benötigt, um durch die gelenkige Anordnung über das Scharnier durch Zusammenklappen der beiden Halbschalen den Pulverinhalator zu bilden, ohne dass es weiterer Bauteile oder Werkzeug bedarf.

Um den Pulverinhalator geeignet handhaben zu können und ihn überall hin mitnehmen zu können, ist dieser erfindungsgemäß aus zwei flachen Halbschalen mit einer länglichen, im Wesentlichen rechteckigen, trapezförmigen, tropfenförmigen oder ovalen Basis ausgebildet. Die Schale ergibt sich, da die Basis durch eine Wandung oder Wandungsabschnitte berandet ist. Die länglich ausgebildeten Halbschalen weisen daher eine Längsachse auf, an deren beiden Enden zum einen die Lufteinlassöffnungen und zum anderen der Auslass vorgesehen sind. Mindestens eine der beiden Halbschalen weist in ihrem von der Auslassseite abgewandten Wandungsabschnitt die Lufteinlassöffnungen auf; vorteilhaft ist es die Halbschale, die nicht die Pulveraufnahmemulde trägt. Dies ist dadurch begründet, dass mit der Gesamtgestaltung der für einen optimalen Strömungsverlauf geformte Fluidpfad ausgebildet werden soll.

"Fluidpfad" meint hierin den Weg, den zunächst die Luft allein und nach Mittragen des Pulvers durch die Luft das Aerosol zurücklegt. Das Aerosol entsteht, wenn der in der Mulde vorliegende, portionierte pulverförmige Wirkstoff durch inhalierte Luft mitgenommen und auf der nachfolgenden Diffusorstrecke hinreichend vermischt und zerstäubt wird. Um im Luftzufuhrbereich einen idealen, vorzugsweise Venturirohrähnlichen Kanal zu bilden, in dem der Fluidpfad lokalisiert ist, weist erfindungsgemäß wenigstens eine der Halbschalen, bevorzugt aber beide, mehrere Leitstege auf, die sich von dem Wandungsabschnitt, an dem die Lufteinlassöffnungen vorliegen und/oder von beiden Wandungsabschnitten, die neben den Lufteinlassöffnungen anschließen, bis hin zu der Pulveraufnahme erstrecken. Dabei reichen die Leitstege einer Halbschale bezüglich ihrer Höhe bis zu der anderen Halbschale oder - wenn beide Halbschalen Leitstege aufweisen, die somit quasi Leitstegpaare bilden - bis zu den Leitstegen der anderen Halbschale, sodass jeweils zwei nebeneinander benachbarte Leitstege (oder benachbarte Leitstegpaare) einen Zuluftkanal ausbilden und den Fluidpfad in dem Lufteinlassbereich begrenzen.

Erfindungsgemäß sind die Leitstege fächerförmig angeordnet, so dass jeder Zuluftkanal einen sich zu der Pulveraufnahmemulde hin verjüngenden Querschnitt aufweist. Weiter ist in jedem Zuluftkanal zumindest eine Luftverwirbelungsstruktur vorgesehen. Die Luft wird durch die trichterförmigen Zuluftkanäle komprimiert und in Torsion versetzt, so dass der durch Einatmen erzeugte Luftstrom eine höchste Geschwindigkeit und Turbulenz an der "Trichterspitze" aufweist, d. h. wenn er die Pulveraufnahmemulde erreicht, die wiederum beidseitig durch Führungswände erfindungsgemäß eingefasst ist, die sich von den äußersten Leitstegen erstrecken und den Luftstrom aus dem Lufteinlassbereich durch die Pulveraufnahmemulde leiten. Durch die turbulente Luftströmung wird das Pulver aus der Pulveraufnahmemulde vollständig mitgenommen. Ein optimaler Strömungsverlauf wird damit unabhängig von der Art und Tiefe der Einatmung des Anwenders sichergestellt.

Um einen in einer Mulde eines Blisterelements enthaltenen Wirkstoff freizusetzen, wenn das Blisterelement mit seiner Mulde in der Pulveraufnahmemulde des Pulverinhalators aufgenommen ist, ist ein Stempel in der Halbschale gegenüber der Pulveraufnahmemulde vorgesehen, der mittels eines elastischen Einsatzes in der Halbschale eingesetzt ist, so dass der Stempel durch Druck von außen an der Stelle des elastischen Einsatzes in Richtung der Pulveraufnahmemulde(n) bewegt werden kann und bei Nachlassen des äußeren Drucks infolge der Rückstellkraft des elastischen Einsatzes wieder in seine ursprüngliche Position zurückkehrt. Der Stempel ist hierbei als Dorn ausgeführt, bspw. als sternförmiger Dorn mit drei, vier oder mehr Kanten, so dass die Wirkstoffmulde des Blisterelements beim Eindrücken des Dorns perforiert wird.

Alternativ oder zusätzlich zu dem Dorn ist an einer der Halbschalen an einem Seitenwandungsabschnitt auf Höhe der Pulveraufnahmemulde eine Aussparung vorgesehen, deren Breite und Höhe der Breite und Höhe eines Blisterelements entsprechen, das zum Einlegen in diesen Pulverinhalator vorbestimmt ist. Der Pulverinhalator an sich kann entsprechend ausgestaltet sein, dass er der Aufnahme bestimmter Blisterelemente dient.

Der erfindungsgemäße Inhalator weist zumindest eins der oben beschriebenen Merkmale des Dorns und der Aussparung auf.

Geeigneter Weise hat die Aussparung eine Höhe, die höher ist als die Höhe des Blisterelements. Hierbei ist allerdings die Höhe der Blisterfolie aus Träger- und Deckelfolie und nicht die Höhe der Medikamentenmulde bzw. Kapsel gemeint. Die zweite Halbschale, die nicht die Aussparung hat, weist an den Seitenwandungsabschnitt, der bei einer zusammengefügten Anordnung der Halbschalen über der Aussparung zu liegen kommt, einen Überstand auf. Dieser ist dazu ausgebildet, die Aussparung zu verschließen, wenn ein entsprechendes Blisterelement in den Pulverinhalator aufgenommen ist, d. h. ein Teil der Blisterfolie durch die Aussparung aus dem Pulverinhalator ragt. Die Aussparung kann so vorgesehen sein, dass der Inhalator für Rechts- oder Linkshänder ausgelegt ist. Durch das Verschließen der Aussparung nach Einlegen des Blisterelements wird verhindert, dass an unerwünschter Stelle Luft beim Inhalieren in den Inhalator eingezogen wird. Der Pulverinhalator kann vorteilhaft als ein Spritzgussbauteil gefertigt werden, was die Herstellung besonders kostengünstig macht. Durch das Filmscharnier können die beiden Bauteile unverlierbar miteinander verbunden bleiben, sie können geöffnet und geschlossen werden, was das Sauberhalten des Pulverinhalators bei Mehrfachnutzung vereinfacht. Zudem unterstützen die Scharniere das richtige Zusammenfügen der Halbschalen durch den Anwender.

Ist eine Ausführungsform des Pulverinhalators vorgesehen, mittels dessen gleichzeitig zwei oder mehr pulverförmige Medikamente inhaliert werden sollen und dementsprechend zwei oder mehr Pulveraufnahmemulden in einer der Halbschalen vorliegen, so sind die Leitstege derart angeordnet, dass jeweils zumindest ein Zuluftkanal zu jeder der Pulveraufnahmemulden führt.

Eine Luftverwirbelungsstruktur kann bspw. von den Leitstegen aus in den Zuluftkanal ragenden Flügeln gebildet werden. Diese Flügel sind bevorzugt an jeweils zwei sich in den Halbschalen gegenüberliegenden und/oder benachbarten Leitstegen alternierend angeordnet und bilden damit Strömungshindernisse für einen entlang dem Fluidpfad strömenden Luftstrom.

Der Auslass des Pulverinhalators kann als Mundstück, etwa als ein Schnabel, oder zur Inhalation durch die Nase als ein Nasenstück, etwa als ein gebogenes Nasenrohr, geformt sein.

Zumindest eine der im Auslassbereich vorgesehenen Desagglomerationsstrukturen kann durch in den Fluidpfad ragende Stege gebildet werden, die sich von gegenüber liegenden Wandungsabschnitten zumindest einer der beiden Halbschalen erstrecken und die bevorzugt quer zum Fluidpfad verlaufen. Besonders bevorzugt sind diese Stege alternierend an den Wandungsabschnitten angeordnet, um für weitere Turbulenzen zu sorgen. Statt der Stege können in dem Auslassbereich auch andere Strömungshindernisse, bspw. radial zum Fluidpfad verlaufende, die etwa schraubenförmig ausgebildet sein können, vorgesehen sein.

Zumindest eine weitere der Desagglomerationsstrukturen kann im Auslassbereich an den Pulverdepositions- und -freigabebereich angrenzen und kann durch einen Umlenksteg oder mehrere solche Strukturen gebildet werden, der bzw. die in einer der Halbschalen, bevorzugt der Halbschale, die die Pulveraufnahmemulde aufweist, angeordnet ist/sind. Die Strömungshindernisse bzw. Desagglomerationsstrukturen dienen dazu, dass beim Transport des Pulvers und der Mitnahme durch den Luftstrom ggf. größere Pulverpartikel durch die Strömungshindernisse, respektive die Stege oder auch schraubenförmigen Lamellen zerschlagen und damit verkleinert werden. Ferner kann dadurch gegebenenfalls der Wirkstoff von einer Trägersubstanz getrennt werden. Es können weitere, beliebig geformte mechanische Widerstände, respektive Strömungswiderstände, als Desagglomeratoren positioniert werden, um den Fluidpfad bedarfsgerecht und der Spezifikation des Medikaments angepasst auszubilden. In Frage kommen hier bspw. auch wallförmige oder pollerförmige Strukturen, die auch ringförmig angeordnet sein können, oder zyklonartige, oder auch als Oval angeordnete Strukturen.

Grundsätzlich kann der Auslass, egal ob er als Mundstück geformt ist oder ob er als Nasenstück weitergebildet wird, einen über seine Gesamtstrecke sich verringernden oder aber auch einen gleichbleibenden Querschnitt aufweisen, der rund oder oval sein kann. Selbstverständlich ist auch ein polygonaler Querschnitt nicht ausgeschlossen, ist aber weniger geeignet. Es bietet sich an, den Querschnitt bis hin zur Austrittsstelle des Aerosols sich verjüngend auszubilden.

Die Gesamtgestaltung des Fluidpfads dient der optimalen Strömung und Beschleunigung des inspirierten Luftstroms und der verbesserten Mitnahme und Zerstäubung des pulverförmigen Wirkstoffs. Es soll insbesondere erreicht werden, dass die Pulveraufnahmemulde von dem darin befindlichen Pulver mit einem Atemzug komplett entleert wird.

Die freie Gestaltung des Profils des Fluidpfads und der entsprechenden Strömungshindernisse bzw. Desagglomeratoren ermöglicht die Anpassung des Inhalators für verschiedene inspirationsgeeignete Wirkstoffe, die insbesondere Arzneimittel sein können. So können trockene, pulverförmige Arzneimittel im Strom in Partikel definierter Größe dispergiert werden.

Der erfindungsgemäße Inhalator ist durch seine Gestaltung und durch die Möglichkeit, ihn aufzuklappen und zu reinigen, besonders hygienisch handhabbar. Er ist ohne Klingen, Federn oder Hebel ausgebildet. Bei der Herstellung und Applikation von Pulveraerosol spielen die Adhäsion und die Reibung im Inhalator eine bedeutende Rolle; Adhäsion und Reibung zwischen Wirkstoff und Inhalator können durch die Turbulenzen erzeugende Fluidpfadführung überwunden werden. Da die Pulverteilchen dazu tendieren, sich an der Inhalatorinnenfläche adhäsiv anzulagern, ist eine geeignete stetige Fluidpfadführung ohne komplette Strömungsumkehrungen daher besonders bevorzugt, um dies zu vermeiden. Die Strömungshindernisse im Luftzufuhrbereich und Auslassbereich sind so gestaltet, Geschwindigkeit und Turbulenz im geführten Luftstrom zu optimieren, um eine bestmögliche Pulverfreisetzung und Desagglomeration zu erreichen, was zu einer verbesserten Inhalationsleistung führt.

Weiter kann die Halbschale, die die Pulveraufnahmemulde aufweist, wenigstens ein Anschlagselement aufweisen, das der Berandung eines zur Aufnahme in den Pulverinhalator vorbestimmten Blisterelementes dient. Damit kann das Blisterelement, wenn es eingelegt ist, nicht verrutschen.

Eine Ausführungsform sieht vor, dass das Öffnen der Wirkstoffmulde des Blisterelements nicht durch Perforation mittels eines beweglichen Dorns, sondern durch Abziehen der Deckelfolie erfolgt. Diese ragt mittels eines umgeschlagenen Überlapps durch die Aussparung aus dem zusammengefügten Pulverinhalator. Damit die Wirkstoffmulde beim Abziehen der Deckelfolie an dem aus dem geschlossenen Pulverinhalator ragenden Überlapp sicher und richtig positioniert in der Pulveraufnahmemulde verbleibt, können beide Halbschalen an den Seitenwandungsabschnitten auf Höhe der Pulveraufnahmemulde gegenüber der Aussparung und dem Überstand eine Riffelung aufweisen, deren Breite in etwa der Breite des zum Einlegen in den Pulverinhalator vorbestimmten Blisterelements entspricht, so dass dieses bzw. ein entsprechender Blisterfolienabschnitt von den geriffelten Seitenwandabschnitten im geschlossenen Zustand des Pulverinhalators festgehalten wird.

Alternativ oder zusätzlich dazu kann vorgesehen sein, an der Halbschale, die die Pulveraufnahme aufweist, als Widerlager einen Überstand, etwa einen kuppelförmigen Überstand, bereitzustellen, der dazu ausgebildet ist, mit einer korrespondierenden Ausnehmung des Blisterelements in Eingriff gebracht zu werden, das dazu vorgesehen ist, in einen erfindungsgemäßen Pulverinhalator eingelegt zu werden.

Schließlich kann der einstückige Pulverinhalator auch aus drei Halbschalen bestehen. Dabei kann die weitere Halbschale über wenigstens ein Scharnier an eine der Halbschalen, vorzugsweise derjenigen ohne Lufteinlässe, angeschnitten, respektive angespritzt sein. Diese Halbschale hat geeigneter Weise ebenfalls eine Basis, die der Grundfläche bzw. Basis der Halbschalen entspricht, die das Strömungsgehäuse bilden. Die dritte angeschnittene Halbschale dient zum einen dazu, den Pulverinhalator angenehm in die Hand einlegen zu können, zum anderen kann die Halbschale bei entsprechender Größe dazu verwendet werden, wenigstens ein Blisterelement darin zu bevorraten. Selbstverständlich kann diese Halbschale auch so ausgebildet sein, dass mehrere geeignete Blisterelemente aufgenommen werden können.

Es ist nicht ausgeschlossen, dass auch noch weitere klappbare Elemente, etwa noch eine weitere Halbschale über entsprechende Scharniere an einer der beschriebenen Halbschalen angelenkt sein können, solange sichergestellt ist, dass die Lufteinlässe nicht verschlossen werden.

Im Bereich der Pulveraufnahmemulde kann wenigstens eine der beiden Halbschalen aus transparentem Kunststoff gefertigt sein, um das Hineinsehen in die Pulveraufnahmemulde zu ermöglichen und es damit zu erlauben, visuell zu überprüfen, ob das Pulver vollständig aus der Pulveraufnahmemulde entleert wurde, nachdem ein Inhalationsvorgang abgeschlossen ist.

An den Halbschalen können geeignete Rastmittel zum Zusammenfügen vorgesehen sein; die Ausbildung derartiger Rastmittel ist dem Fachmann bekannt. Die Rastmittel können auf den entsprechenden Rändern der Halbschalen vorgesehen sein. Geeigneter Weise verfügen alle der Halbschalen, es können zwei, drei, aber auch durchaus vier sein, über Rastmittel, so dass diese durch Zusammenklappen einfach miteinander verrastet und gekoppelt werden können.

Weiterer Gegenstand der Erfindung ist ein Pulverinhalationsset, das aus dem erfindungsgemäßen Pulverinhalator einerseits und zum anderen aus einem entsprechenden Blisterelement besteht, das zur Aufnahme in den Pulverinhalator dimensioniert ist. Ein solches Blisterelement hat eine Trägerplatte oder -folie mit einer Mulde, in der das Pulver aufgenommen ist, respektive der inhalierbare Wirkstoff. Ein passendes Blisterelement kann aber auch zwei oder mehr Mulden mit unterschiedlichen, gleichzeitig zu inhalierenden Wirkstoffpulvern aufweisen. Das Blisterelement verfügt ferner über eine Deckelplatte oder -folie, die die Mulde(n) solange verschließt, wie das Pulver in den Blister bevorratet werden soll. Die Mulde(n) des Blisterelements ist/sind in Größe und Position entsprechend zur Aufnahme in die Pulveraufnahmemulde(n) des Pulverinhalators ausgebildet. Um durch Öffnen der Mulde des Blisterelements das Pulver freizusetzen, wenn der Inhalationsvorgang durchgeführt werden soll, kann die Deckelplatte von der Trägerplatte und damit von der Wirkstoffmulde abgelöst werden, indem die Deckelplatte bspw. mit einem umklappbaren Überlapp ausgebildet ist, der bei in den Pulverinhalator eingelegtem Blisterelement durch die Aussparung in einem Seitenwandungsabschnitt auf Höhe der Pulveraufnahmemulde aus dem Pulverinhalator herausragt und abgezogen werden kann. Wie oben ausgeführt, kann zum Abziehen der Deckelfolie als Widerlager eine Riffelung an den Seitenwandabschnitten gegenüber der Aussparung oder ein kuppelförmiger Überstand vorgesehen sein; je nach dem wird ein Blisterelement mit verlängerter Träger- und Deckelfolie eingesetzt, das bei zusammengeklapptem Pulverinhalator zwischen der Riffelung gehalten wird, oder das Blisterelement weist eine Ausnehmung auf, die in Bezug zu der Wirkstoffmulde entsprechend dem kuppelförmigen Überstand in Bezug zur Pulveraufnahmemulde positioniert ist.

Eine alternative Ausführungsform sieht ein einfacheres Blisterelement vor, das nicht mit einem Abschnitt der Deckelplatte aus dem Pulverinhalator ragen muss, sondern lediglich passgenau mit der Wirkstoffmulde in die Pulveraufnahmemulde des Pulverinhalators eingelegt wird. Gegebenenfalls kann in dieser Ausführungsform auch ein kapselartiges Blisterelement oder eine speziell ausgebildete Kapsel eingesetzt werden. Das Öffnen der Wirkstoffmulde erfolgt hierbei nicht durch Abziehen der Deckelplatte sondern durch Perforation derselben. Ein dafür konzipierter erfindungsgemäßer Pulverinhalator ist mit einem elastisch bewegbaren und als Dorn ausgebildeten Stempel gegenüber der Pulveraufnahmemulde ausgebildet.

Es kann auch vorgesehen sein, dass ein erfindungsgemäßer Pulverinhalator mit beiden Öffnungsmechanismen ausgestattet ist, so dass beide Blisterarten geöffnet werden können.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Dabei zeigen:
- Fig. 1: perspektivische Ansichten erfindungsgemäßer Pulverinhalatoren für inhalative Applikation durch den Mund in geöffnetem Zustand, mit
a) einer Riffelung und
b) einem kuppelförmigen Überstand als Widerlager,
- Fig. 2: Ansichten entsprechend Fig. 1a) und b) mit eingelegtem Blister,
- Fig. 3: eine perspektivische Ansicht des Pulverinhalators aus Fig. 1 in geschlossenem Zustand,
- Fig. 4: eine perspektivische Längsschnittansicht durch den Pulverinhalator aus Fig. 3,
- Fig. 5: eine Längsschnittansicht durch den geschlossenen Pulverinhalator ohne Blister,
- Fig. 6: eine Längsschnittansicht durch einen weiteren erfindungsgemäßen Pulverinhalator zur nasalen Applikation in geschlossenem Zustand ohne Blister,
- Fig. 7: eine perspektivische Ansicht des Pulverinhalator aus Fig. 6,
- Fig. 8: eine perspektivische Ansicht des Pulverinhalators aus Fig. 6/7 in geöffnetem Zustand,
- Fig. 9: eine Draufsicht auf den Pulverinhalator aus Fig. 6 bis 8.

Die erfindungsgemäße Vorrichtung bezieht sich auf einen Pulverinhalator zur inhalativen Applikation durch Mund oder Nase eines in einem Blister bevorrateten pulverförmigen Wirkstoffs, der ein Medikament sein kann, aber auch ein Wirkstoff, der nicht zwingend als Medikament definiert ist, und der inhalativ von einer Person aufgenommen wird.

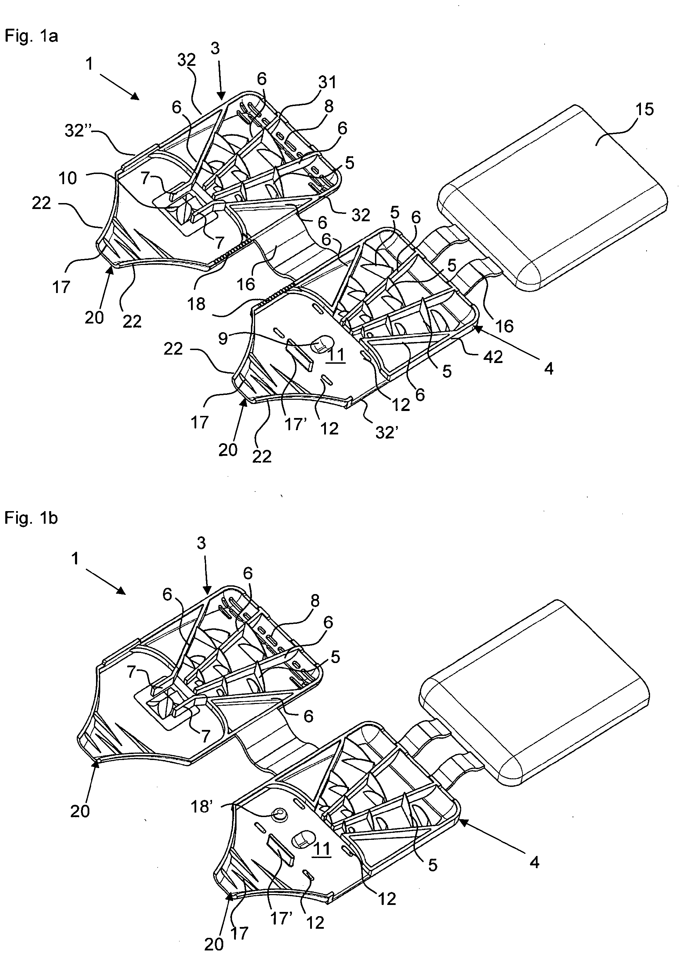

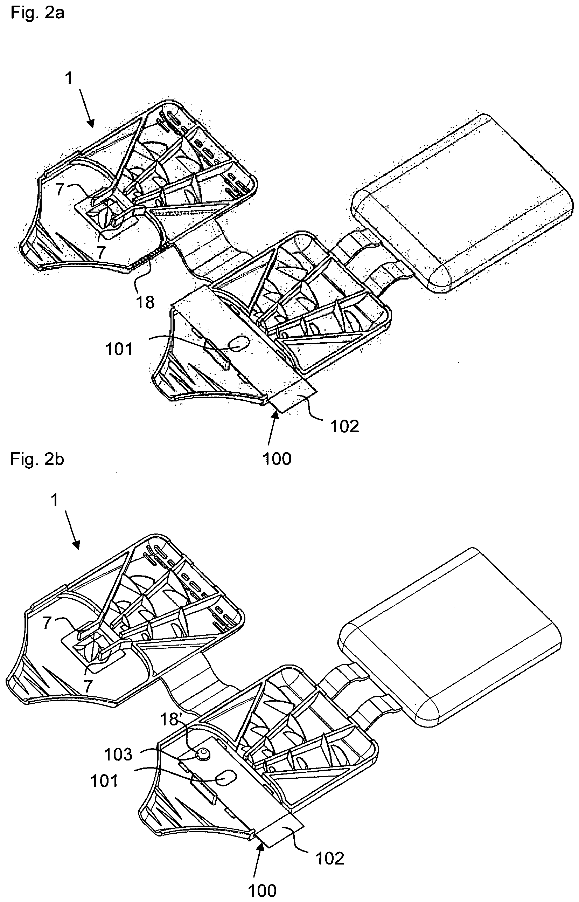

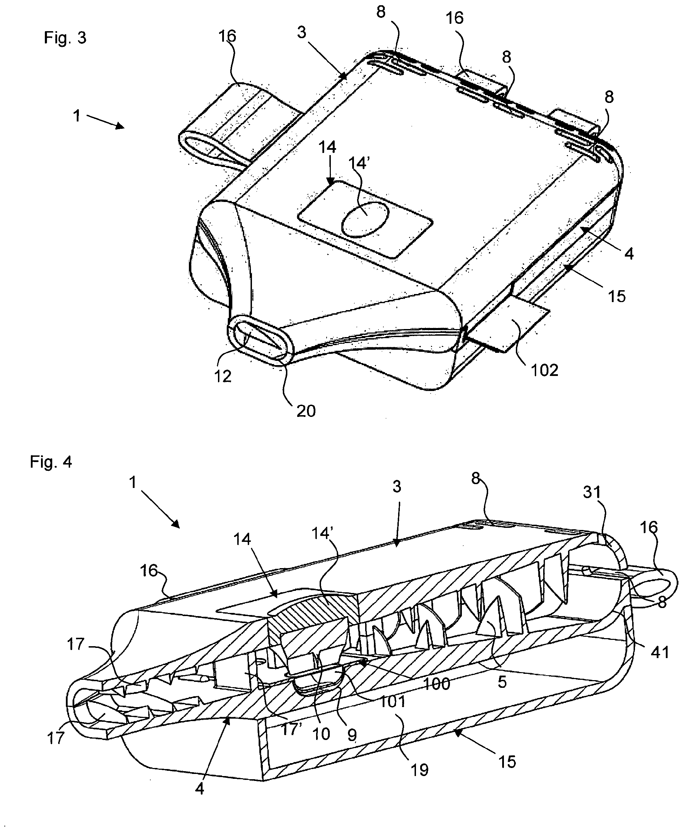

**Fig. 1a****) und b)** zeigen erfindungsgemäße Pulverinhalatoren 1 zur inhalativen Applikation in geöffnetem Zustand, bestehend aus zwei Halbschalen 3 und 4, die in zusammengeklapptem Zustand einen betriebsbereiten Pulverinhalator bilden. Die beiden Pulverinhalatoren 1 unterscheiden sich lediglich hinsichtlich der Ausführung des Widerlagers zum Halten des Blisterelements beim Abziehen der Deckelfolie. In der dargestellten Form kann der Pulverinhalator 1 bspw. vorteilhaft rasch und günstig mit einem einzigen Werkzeug durch Spritzgießen eines Kunststoffes einstückig hergestellt werden. Um den Pulverinhalator 1 in betriebsbereiten Zustand zu versetzen, wird an einer dafür vorgesehenen Stelle das Blisterelement 100 eingelegt (**siehe** **Fig. 2a****,b**). Nach Zusammenklappen der beiden Halbschalen 3 und 4 und der Öffnung der Medikamentenmulde 101 des Blisterelements 100 ist der Pulverinhalator 1 einsatzbereit (**Fig. 3**).

Der Pulverinhalator 1 lässt sich grob in drei Teilbereiche gliedern:
- den Auslassbereich mit Desagglomerationsstrukturen 17,17', der in dem mit Fig. 1 bis 5 dargestellten Beispiel als Mundstück 20 ausgeformt ist,
- den Luftzufuhrbereich, der sich von der mit Lufteinlässen 8 versehenen Rückwand 31, also der Wandung, die an der Basis der Halbschale 3 abgewandt von dem Auslassbereich vorliegt, bis zur Blisterkammer 11 erstreckt, und der die durch die Leitstege 6 getrennten, trichterförmigen Zuluftkanäle aufweist, und
- den Pulverdepositions- und -freisetzungsbereich, der die Blisterkammer 11 mit der Pulveraufnahmemulde 9 umfasst, in die ein Blisterelement 100 mit einer Wirkstoffmulde 101 aufgenommen werden kann.

Die erfindungsgemäße Gestaltung der drei Teilbereiche sorgt dafür, dass eine zur Applikation des Wirkstoffs, hierin alternativ auch als Medikament bezeichnet, beim Einatmen optimale Luftströmung gebildet wird, unabhängig davon, in welcher Weise der Patient einatmet. Auf diese Weise kann das Medikament optimal inhaliert werden und damit bestmögliche Wirkung zeigen.

Der Luftzufuhrbereich wird bei den in den Figuren gezeigten Beispielen durch drei sich zu der Pulveraufnahmemulde 9 hin verjüngende, durch die Leitstege 6 begrenzte Zuluftkanäle gebildet, in die durch die Lufteinlässe 8 Luft einströmt, wenn der Patient mit dem in den Mund aufgenommenen Mundstück 20 einatmet. Die Lufteinlässe 8 sind in der Rückwand 31 der Halbschale 3 sowie an der Kontaktfläche der Rückwände 31,41 zwischen beiden Halbschalen 3,4 ausgebildet (vgl. **Fig. 4**). In jedem Zuluftkanal sind Luftverwirbelungsstrukturen in Form von Flügeln 5 (Umlenkflügel 5) angeordnet, die sich jeweils von den Leitstegen 6 beider Halbschalen 3,4 in die Kanäle erstrecken. Die sich verjüngenden Zuluftkanäle und die Umlenkflügel 5 sorgen für Turbulenz und erhöhen die Geschwindigkeit des Zuluftstroms vor Eintritt in die Pulveraufnahmemulde 9.

Natürlich kann die Anzahl der Zuluftkanäle in anderen Ausführungsformen, insbesondere auch in Ausführungsformen mit mehr als einer Pulveraufnahmemulde, variiert werden.

Zur Anordnung des Blisters 100 (vgl. **Fig. 2a****) und b**) weist die Halbschale 4 in einer Seitenwand 42 eine Aussparung 32' auf, durch die sich der eingelegte Blister 100 mit einem umgeschlagenen Überlapp 102 der Deckelfolie des Blisterelements 100 erstreckt. Luftdicht verschlossen wird diese Aussparung 32' durch den Überstand 32" an der anderen Halbschale 3. Die Wirkstoffmulde 101 des Blisterelements 100, die man auch als Kapsel bezeichnen könnte, findet in der in die Halbschale 4 eingeformten Mulde 9 Aufnahme. Während **Fig. 1a****) und** **2a****)** eine Ausführungsform zeigen, in denen das Blisterelement 100 durch die Riffelungen 18 in den Seitenwänden 32,42 gegenüber der Aussparung 32' und dem Überstand 32" in den Halbschalen 3,4 festgehalten wird, wenn der Pulverinhalator 1 geschlossen wird, wird das Widerlager des Pulverinhalators 1 in **Fig. 1b****) und** **2b****)** durch einen neben der Pulveraufnahmemulde 9 platzierten kuppelförmigen Überstand 18' gebildet, der eine entsprechend positionierte Öffnung 103 des Blisterelements 100 durchdringt und für einen unverrückbaren Halt des Blisterelements 20 in dem Pulverinhalator 1 sorgt, wenn zum Öffnen der Wirkstoffmulde 101 an dem aus dem Pulverinhalator 1 ragenden Abschnitt 102 der Deckelfolie des Blisterelements 100 (vgl. **Fig. 3**) gezogen wird.

Zur positionsgenauen Anordnung des Blisterelements 100 sind ferner die Anschläge 12 in der Halbschale 4 vorgesehen. Weiter unterstützt der als Desagglomerator fungierende Umlenksteg 17' die Positionierung des Blisterelements 100.

Zur Führung der Luftströmung durch die in die Pulveraufnahmemulde 9 eingelegte und geöffnete Wirkstoffmulde 101 sind in der Halbschale 3 an entsprechender Stelle Führungswände 7 **(****Fig. 1****)** vorgesehen, die sich von den äußersten Leitstegen 6 und im geschlossenen Pulverinhalator 1 bis zum Blisterelement 100 erstrecken, so dass der Luftstrom aus dem Lufteinlassbereich durch die Pulveraufnahmemulde 9 bzw. die darin angeordnete geöffnete, mit Pulver gefüllte Mulde 101 des Blisterelements 100 geleitet wird.

An die Blisterkammer 11 schließt sich der Auslassbereich an, durch den das nun gebildete Aerosol aus Luft und Medikamentenpulver ausgelassen wird. Die Blisterkammer 11 wird zum Auslassbereich hin durch den Desagglomerator 17' begrenzt, der zusammen mit weiteren Desagglomeratoren 17 den nun mit Wirkstoff beladenen Luftstrom nicht direkt zum Auslass gelangen lässt, indem durch Umlenkungen Turbulenzen erzeugt werden, um das Pulver zu desagglomerieren und um erneutes Absetzen oder Agglomerieren des Pulvermedikaments zu verhindern. Allerdings sorgt die Querschnittsaufweitung nach der durch die Führungswände 7 eng begrenzten Passage durch die Pulveraufnahmemulde vor der Umlenkung durch Desagglomerator 17' zunächst für eine Reduktion der Strömungsgeschwindigkeit und erzielt damit einen Spacer-Effekt, d. h. die Wirkstoffpartikel werden gleichmäßig im Luftstrom verteilt. Anschließend wird die Desagglomeration der Wirkstoffpartikel durch in den Fluidpfad kragende, flügelartige Stege 17 als Desagglomeratoren in dem sich verjüngenden Mundstück 20 unterstützt. Generell können Desagglomeratoren auch mit anderen Strukturen als den hier dargestellten Stegen 17,17' ausgeformt sein, bspw. als Cyclone oder Poller, solange die Strukturen für eine Verwirbelung des Luftstroms nach Passage der Pulveraufnahmemulde sorgen.

Vorteilhaft einfach lässt sich der Pulverinhalator 1 aus den zwei Halbschalen 3, 4, die über ein Filmscharnier 16 verbunden sind, nach Einlegen eines Blisterelements 100 während des geöffneten Zustands durch Zusammenklappen in betriebsbereiten Zustand überführen. Durch die flache Bauweise kann der Pulverinhalator 1 einfach und bequem mitgeführt werden, ohne dass Taschen an der Kleidung übermäßig ausgebeult werden. Wird die Einnahme des Medikaments erforderlich, ist nur noch das Abziehen des Endabschnitts 102 zur Öffnung der Pulvermulde 101 bzw. das Durchstechen der Deckelfolie erforderlich, und das Medikament kann inhaliert werden.

Um in einfacher Weise weitere Blister mitführen zu können, ist in dem vorliegenden Beispiel vorgesehen, dass eine weitere Halbschale 15 über Filmscharniere 16 an die Halbschale 4 angelenkt ist. Generell ist es natürlich auch möglich, dass diese Vorratshalbschale 15 auch an der Halbschale 3 angeordnet ist; in diesem Fall sollten die Lufteinlässe 8, die im gezeigten Beispiel an der Rückwand 31 der Halbschale 3 vorliegen, in der anderen Halbschale 4 angeordnet sein. Wird die Vorratshalbschale 15 an die Halbschale 4 eingeschwenkt, so wird, wie in **Fig. 4** zu sehen ist, ein Vorratsraum 19 begrenzt, in dem z. B. weitere Blister mitgeführt werden können. Ferner kann die Griffergonomie beim Halten des Pulverinhalators 1 durch die Vorratshalbschale 15 verbessert werden.

In einer nicht gezeigten Ausführungsform kann an Stelle einer dritten Halbschale eine Verwirbelungsstruktur mittels Scharnier bzw. können mehrere Verwirbelungsstrukturen angelenkt sein, die durch Einklappen in dem/den Luftkanal/Luftkanälen platziert werden kann/können.

Wie in **Fig. 1 bis 5** zu sehen ist, kann ein zum Abziehen der Deckelfolie alternativer Öffnungsmechanismus für die Wirkstoffmulde 101 eines Blisterelements 100 vorgesehen sein, der einen als Dorn ausgebildeten Stempel 10 aufweist. Der Dorn 10 ist durch einen elastischen Einsatz 14 in der Halbschale 3 gehalten, der eine Wölbung 14' nach außen aufweist, die dem Anwender den Druckpunkt zeigt, der betätigt werden muss, um die Deckelfolie der Blistermulde 101 des eingelegten Blisterelements 100 mit dem Dorn 10 zu durchdringen. Nach Betätigung des Druckpunktes durch den Anwender wird der Dorn 10 durch den elastischen Einsatz 14 wieder in seine Anfangsposition zurückgestellt. Vorteilhafterweise leitet der Stempel 10 in dieser Stellung die turbulente Luftströmung aus dem Lufteinlassbereich in die Pulveraufnahmemulde 9 bzw. die darin aufgenommene Wirkstoffmulde 101. Die Herstellung dieses Pulverinhalators kann erfolgen, indem zunächst der elastische Einsatz 14 gespritzt wird und die restliche Inhalatorform an den elastischen Einsatz 14 angespritzt wird oder umgekehrt, bspw. in einem 2K-Spritzgussverfahren.

Es ist möglich, dass ein erfindungsgemäßer Pulverinhalator beide Öffnungsmechanismen, d. h. einen Stempel und ein Widerlager, bestehend aus Riffelungen oder Poller, aufweist, um verschiedene Blisterarten öffnen zu können, es kann aber auch genügen, wenn ein erfindungsgemäßer Pulverinhalator nur eine der Alternativen aufweist.

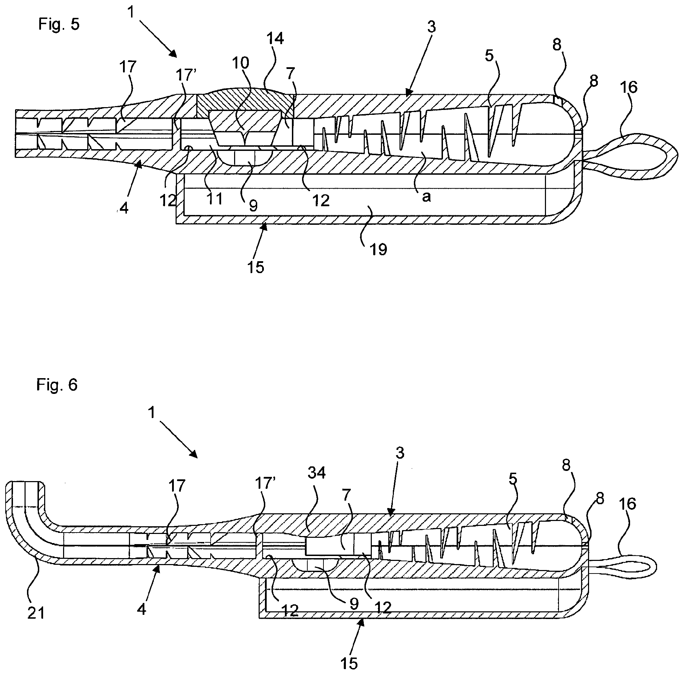

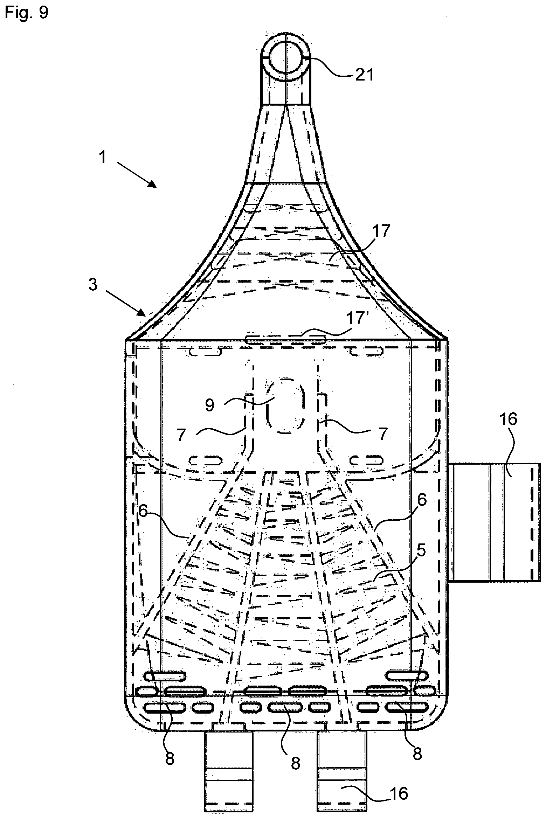

Wie in der Seitenschnittansicht in **Fig. 5** zu sehen ist, verjüngen sich die Luftzufuhrkanäle nicht nur in der Breite sondern auch in der Höhe in Richtung der Blisterkammer 11, wobei die Wandstärke der Halbschalen 3 und 4 in entsprechender Weise zunimmt. Die in die Zuluftkanäle ragenden Flügel 5 sorgen für einen schnecken- oder spiralförmigen Weg, der die Luftströmung in Torsion versetzt (ähnlich einem Tornado). Durch die Drehung werden Fliehkräfte und Druckphänomene erzeugt, die wichtig für das vollständige Mitreißen des Pulvers aus der Pulveraufnahmemulde 9 sind. Besonders effektiv sind dabei gegenläufige Wirbel, wie sie bspw. durch den mittleren und rechten Zuluftkanal in **Fig. 9** erhalten werden können.

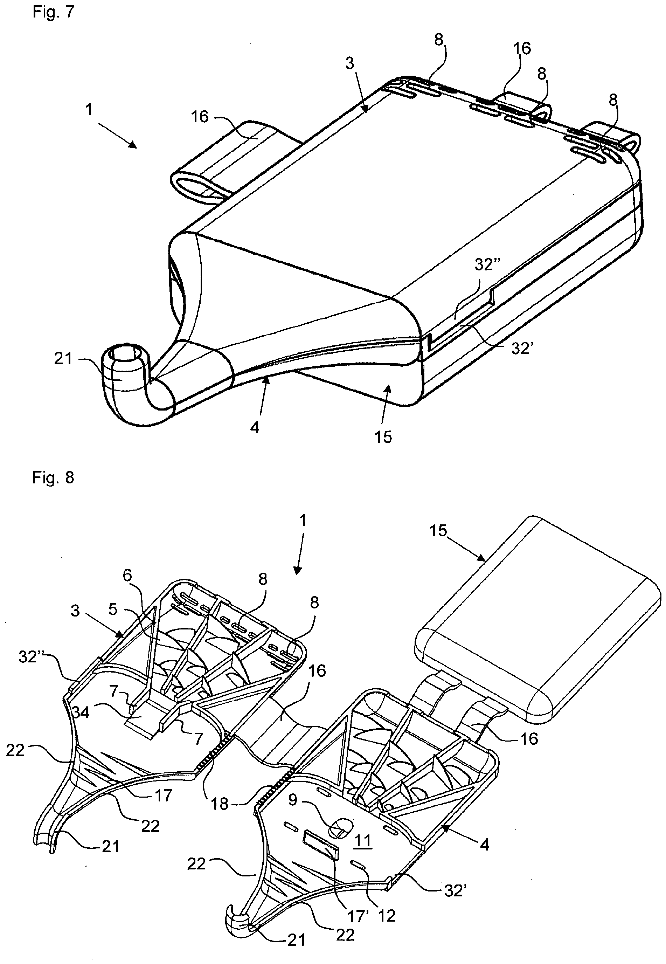

Die weiteren **Figuren 6 bis 9** stellen eine weitere beispielhafte Ausführungsform des erfindungsgemäßen Pulverinhalators 1 dar, der für nasale Applikation vorgesehen ist. Daher ist der Auslass als gebogenes Nasenrohr 21 ausgeformt. Im Übrigen entspricht dieser Pulverinhalator 1 für nasale Applikation weithingehend in Aufbau und Funktionsweise dem zuvor beschriebenen Pulverinhalator 1 für inhalative Applikation durch den Mund, allerdings ohne den beweglichen Stempel. In diesem Pulverinhalator erfolgt das Öffnen der Wirkstoffmulde des Blisterelements also durch Abziehen der Deckelplatte. Gegenüber der Pulveraufnahmemulde 9 weist die Halbschale 3 eine Wandverdickung 34 zwischen den Führungswänden 7 auf, um an dieser Stelle eine Querschnittszunahme zu vermeiden und den turbulenten Luftstrom aus dem Luftzufuhrbereich in die Pulveraufnahmemulde 9 bzw. die darin aufgenommene Wirkstoffmulde zu leiten.

Selbstverständlich kann auch ein Pulverinhalator mit Nasenstück mit dem elastisch gelagerten Stempel zur Perforation des Blisters ausgeführt sein. Generell kann auf die als Widerlager beim Abziehen dienenden Elemente (Riffelung oder Überstand) sowie die Aussparung und den zugehörigen Überstand in den Seitenwänden verzichtet werden, wenn nur der Stempel zum Öffnen der Blister eingesetzt werden soll.

Schließlich wird nicht ausgeschlossen, dass ein erfindungsgemäßer Pulverinhalator auch ohne Blisterelement eingesetzt werden kann, indem im geöffneten Zustand des Pulverinhalators direkt eine Pulverdosis in die Pulveraufnahmemulde gegeben wird, die sofort nach Zusammenklappen der Halbschalen inhaliert wird.

## Patentansprüche

1. Pulverinhalator (1), umfassend
- zwei Halbschalen (3,4), die durch zumindest ein Filmscharnier (16) miteinander verbunden und einstückig ausgebildet sind, wobei beide Halbschalen (3,4) in einer zusammengefügten Anordnung einen Lufteinlassbereich, einen Pulverdepositions- und -freigabebereich und einen Auslassbereich umschließen, durch die ein Fluidpfad verläuft, **wobei**
- zumindest eine der Halbschalen (3,4) in dem Lufteinlassbereich zumindest eine Lufteinlassöffnung (8) aufweist,
- in dem Pulverdepositions- und -freigabebereich eine der Halbschalen (3,4) zumindest eine Pulveraufnahmemulde (9) aufweist, in der eine Mulde (101) eines zur Aufnahme in dem Pulverinhalator (1) dimensionierten Blisterelements (100) aufnehmbar ist, das eine Trägerfolie aufweist, die zumindest die Mulde (101) aufweist, die zur Aufnahme zumindest eines inhalierbaren Wirkstoffpulvers ausgebildet ist, wobei eine Deckelplatte des Blisterelements (100) die Mulde (101) verschließt, die in Größe und Position entsprechend zur Aufnahme in der zumindest einen Pulveraufnahmemulde (9) des Pulverinhalators (1) ausgebildet ist,
- der Auslassbereich zumindest eine Desagglomerationsstruktur (17,17') und einen Auslass für Aerosol aufweist, der durch die Halbschalen (3,4) ausgebildet wird,
- die zwei Halbschalen (3,4) flach sind und eine längliche im Wesentlichen rechteckige, trapezförmige, tropfenförmige oder ovale Basis aufweisen, die durch eine Wandung oder Wandungsabschnitte berandet ist,
- die zumindest eine Lufteinlassöffnung (8) an einem von dem Auslass abgewandten Wandungsabschnitt (31,41) angeordnet ist,
- an zumindest einer der zwei Halbschalen (3,4) zumindest zwei, bevorzugt mehrere Leitstege (6) vorliegen, die sich von dem von dem Auslass abgewandten Wandungsabschnitt (31,41) und/oder von zu dem von dem Auslass abgewandten Wandungsabschnitt (31, 41) benachbarten Seitenwandungsabschnitten (32,42) bis hin zu der zumindest einen Pulveraufnahmemulde (9) erstrecken, wobei sich die Leitstege (6) der zumindest einen Halbschale (3,4) in ihrer Höhe bis zu der anderen Halbschale (3,4) oder bis zu den Leitstegen (6) der anderen Halbschale (3,4) erstrecken, wobei jeweils zwei nebeneinander benachbarte Leitstege (6) einen Zuluftkanal ausbilden und den Fluidpfad in dem Lufteinlassbereich begrenzen,
- die nebeneinander benachbarten Leitstege (6) fächerförmig angeordnet sind,
- jeder Zuluftkanal einen sich zu der zumindest einen Pulveraufnahmemulde (9) hin verjüngenden Querschnitt aufweist,
- die zumindest eine Pulveraufnahmemulde (9) entlang dem Fluidpfad beidseitig durch Führungswände (7) eingefasst ist, die sich von den äußersten zu der zumindest einen Pulvermulde (9) führenden Leitstegen (6) erstrecken,
- in jedem Zuluftkanal zumindest eine Luftverwirbelungsstruktur angeordnet ist, und wobei der Pulverinhalator (1) mindestens eins der folgenden Merkmale A und B aufweist:
A. in dem Pulverdepositions- und -freigabebereich weist die Halbschale (3,4), die nicht die zumindest eine Pulveraufnahmemulde (9) aufweist, an einer der zumindest einen Pulveraufnahmemulde (9) gegenüberliegenden Stelle einen Stempel (10) auf, der mittels eines elastischen Einsatzes (14) in dieser Halbschale (3,4) in Richtung der zumindest einen Pulveraufnahmemulde (9) bewegbar eingesetzt ist, wobei der Stempel (10) als Dorn zur Öffnung einer Wirkstoffmulde (101) eines in den Pulverinhalator (1) eingelegten Blisterelements (100) ausgebildet ist; und
B. an einer der Halbschalen (3,4) weist einer der Seitenwandungsabschnitte (32,42) auf Höhe der Pulveraufnahmemulde (9) eine Aussparung (32') auf, deren Breite und Höhe einer Breite und Höhe eines zum Einlegen in den Pulverinhalator (1) vorbestimmten Blisterelements (100) entsprechen, wobei die zweite Halbschale (3,4), die nicht die Aussparung (32') hat, an dem Seitenwandungsabschnitt (32,42), der bei einer zusammengefügten Anordnung der Halbschalen (3,4) über der Aussparung (32') zu liegen kommt, einen Überstand (32") aufweist, der dazu ausgebildet ist, die Aussparung (32') zu verschließen, wenn ein Blisterelement (100) in dem Pulverinhalator (1) aufgenommen ist.

2. Pulverinhalator (1) nach Anspruch 1, **dadurch gekennzeichnet, dass** der Pulverinhalator (1) ein Spritzgussbauteil ist.

3. Pulverinhalator (1) nach zumindest Anspruch 1 oder 2, **dadurch gekennzeichnet, dass** der Pulverinhalator (1) zumindest zwei Pulveraufnahmemulden (9) aufweist, wobei jeweils zumindest ein Zuluftkanal zu jeder der Pulveraufnahmemulden (9) führt.

4. Pulverinhalator (1) nach einem der Ansprüche 1 bis 3,
**dadurch gekennzeichnet, dass**
die zumindest eine Luftverwirbelungsstruktur durch mehrere von den Leitstegen (6) in den jeweiligen Zuluftkanal ragende Flügel (5) gebildet wird.

5. Pulverinhalator (1) nach Anspruch 4,
**dadurch gekennzeichnet, dass**
die Flügel (5) an jeweils zwei sich gegenüberliegenden und/oder benachbarten Leitstegen (6) alternierend angeordnete Flügel (5) sind und Schikanen für einen entlang dem Fluidpfad strömenden Luftstrom bilden.

6. Pulverinhalator (1) nach einem der Ansprüche 1 bis 5,
**dadurch gekennzeichnet, dass**
der Auslass als ein Mundstück oder ein Nasenstück geformt ist.

7. Pulverinhalator (1) nach Anspruch 6,
**dadurch gekennzeichnet, dass**)
das Mundstück als ein Schnabel (20) oder das Nasenstück als ein gebogenes Nasenrohr (21) geformt ist.

8. Pulverinhalator (1) nach einem der Ansprüche 1 bis 7,
**dadurch gekennzeichnet, dass**
zumindest eine der Desagglomerationsstrukturen (17) im Auslassbereich der beiden Halbschalen (3,4) durch an den sich gegenüberliegenden Wandungsabschnitten (22) in den Fluidpfad ragende Stege (17) gebildet wird.

9. Pulverinhalator (1) nach Anspruch 8,
**dadurch gekennzeichnet, dass**
die in den Fluidpfad ragenden Stege (17) alternierend an den beiden Wandungsabschnitten (22) angeordnet sind.

10. Pulverinhalator (1) nach zumindest einem der Ansprüche 1 bis 9,
**dadurch gekennzeichnet, dass**
zumindest eine der Desagglomerationsstrukturen (17') im Auslassbereich an den Pulverdepositions- und -freigabebereich angrenzt und durch zumindest einen Umlenksteg (17') gebildet wird, der in einer der Halbschalen (3,4) angeordnet ist.

11. Pulverinhalator (1) nach Anspruch 10,
**dadurch gekennzeichnet, dass**
der zumindest eine Umlenksteg (17') in der Halbschale (3,4), die die Pulveraufnahmemulde (9) aufweist, angeordnet ist.

12. Pulverinhalator (1) nach einem der vorstehenden Ansprüche,
**dadurch gekennzeichnet, dass**
die Halbschale (3,4), die die Pulveraufnahmemulde (9) aufweist, zumindest einen Anschlag (12) aufweist, der zur Berandung des zur Aufnahme in dem Pulverinhalator (1) vorbestimmten Blisterelements (100) angeordnet ist.

13. Pulverinhalator (1) nach einem der vorstehenden Ansprüche,
**dadurch gekennzeichnet, dass**
beide Halbschalen (3,4) an den Seitenwandungsabschnitten (32,42) auf Höhe der Pulveraufnahmemulde (9) gegenüber der Aussparung (32') und dem Überstand (32") eine Riffelung (18) aufweisen.

14. Pulverinhalator (1) nach einem der vorstehenden Ansprüche,
**dadurch gekennzeichnet, dass**
die Halbschale (3,4), die die Pulveraufnahmemulde (9) aufweist, einen Überstand aufweist, der dazu ausgebildet ist, mit einer korrespondierenden Ausnehmung (103) des zur Aufnahme in dem Pulverinhalator (1) vorbestimmten Blisterelements (100) in Eingriff zu kommen.

15. Pulverinhalator (1) nach Anspruch 14,
**dadurch gekennzeichnet, dass**
der Überstand ein kuppelförmiger Überstand (18') ist.

16. Pulverinhalator (1) nach einem der vorstehenden Ansprüche,
**dadurch gekennzeichnet, dass**
zumindest eine weitere Halbschale (15) über zumindest ein Scharnier (16) an eine der Halbschalen (3,4)angeschnitten ist.

17. Pulverinhalator (1) nach Anspruch 16,
**dadurch gekennzeichnet, dass**
die zumindest eine weitere Halbschale (15) an die Halbschale (3,4) ohne Lufteinlässe (8) angeschnitten ist.

18. Pulverinhalator (1) nach Anspruch 16 oder 17,
**dadurch gekennzeichnet, dass**
die weitere Halbschale (15) eine Basis aufweist, die der Basis der weiteren Halbschalen (3,4) entspricht und die eine Größe zur Bevorratung zumindest eines zur Aufnahme in dem Pulverinhalator (1) vorbestimmten Blisterelements (100) aufweist.

19. Pulverinhalator (1) nach einem der vorstehenden Ansprüche,
**dadurch gekennzeichnet, dass**
an den Halbschalen (3,4,15) Rastmittel zum Zusammenfügen zumindest der ersten Halbschale (3) mit der zweiten Halbschale (4) vorgesehen sind.

20. Pulverinhalator (1) nach einem der Ansprüche 16 bis 19,
**dadurch gekennzeichnet, dass**
an der zumindest einen weiteren Halbschale (15) Rastmittel zum Zusammenfügen mit einer der Halbschalen (3,4,15) vorgesehen sind.

21. Pulverinhalationsset (1,100)
**dadurch gekennzeichnet, dass**
es einen Pulverinhalator (1) nach zumindest einem der Ansprüche 1 bis 20 und ein zur Aufnahme in dem Pulverinhalator (1) dimensioniertes Blisterelement (100) aufweist, wobei das Blisterelement (100) eine Trägerfolie mit zumindest einer Mulde (101) aufweist, die zur Aufnahme zumindest eines inhalierbaren Wirkstoffpulvers ausgebildet ist, und eine Deckelplatte, die die Mulde (101) verschließt, wobei die zumindest eine Mulde (101) des Blisterelements (100) in Größe und Position entsprechend zur Aufnahme in der zumindest einen Pulveraufnahmemulde (9) des Pulverinhalators (1) ausgebildet ist.

22. Pulverinhalationsset (1,100) nach Anspruch 21,
**dadurch gekennzeichnet, dass**
die Deckelplatte zum Öffnen der Mulde (101) von der Trägerplatte durch eine Aussparung (32') in einem Seitenwandungsabschnitt (32,42) auf Höhe der Pulveraufnahmemulde (9) des Pulverinhalators (1) ablösbar ausgebildet ist,
und/oder
die Deckelplatte zum Öffnen der Mulde (101) durch einen gegenüber der Pulveraufnahmemulde (9) angeordneten, elastisch bewegbaren und als Dorn ausgebildeten Stempel (10) durchdringbar ist.

## Claims

1. Powder inhaler (1), comprising
- two half shells (3, 4) that are connected to each other by at least one film hinge (16) and are formed as one piece, wherein both half shells (3, 4) in a joined arrangement enclose an air inlet area, a powder deposition and release area, and an outlet area through which a fluid path is extending, **wherein**
- at least one of the half shells (3, 4) in the air inlet area comprises at least one air inlet opening (8)
- in the powder deposition and release area, one of the half shells (3, 4) comprises at least one powder receiving depression (9), in which a depression (101) of a blister element (100) dimensioned for reception in the powder inhaler (1) can be received, which comprises a support film comprising at least the depression (101) that is formed for receiving at least one active ingredient powder that is inhalable, wherein a cover plate closes off the depression (101), that in size and position is formed correspondingly for reception in the at least one powder receiving depression (9) of the powder inhaler (1),
- the outlet area comprises at least one de-agglomeration structure (17, 17') and an outlet for aerosol that is formed by the half shells (3, 4),
- the two half shells (3, 4) are flat and comprise an elongate substantially rectangular, trapezoidal, drop-shaped or oval base that is rimmed by a wall or wall sections,
- the at least one air inlet opening (8) is arranged on a wall section (31, 41) facing away from the outlet,
- on at least one of the two half shells (3, 4) at least two, preferably several, guide webs (6) are present which are extending from the wall section (31, 41) facing away from the outlet and/or from sidewall sections (32, 42) adjacent to the wall section (31, 41) facing away from the outlet all the way to the at least one powder receiving depression (9), wherein the guide webs (6) of the at least one half shell (3, 4) in regard to their height extend all the way to the other half shell (3, 4) or to the guide webs (6) of the other half shell (3, 4), wherein two side by side neighboring guide webs (6) form an air supply channel and delimit the fluid path in the air inlet area, respectively,
- the side by side neighboring guide webs (6) are arranged in a fan-shaped manner
- each air supply channel comprises a cross-section that is tapering toward the at least one powder receiving depression (9), and
- in each one of the air supply channels at least one air swirling structure is arranged,
and wherein the powder inhaler (1) comprises at least one of the following features A and B:
A. in the powder deposition and release area the half shell (3, 4) that does not comprise the at least one powder receiving depression (9) comprises, at a location which is positioned opposite the at least one powder receiving depression (9), a plunger (10) that is elastically moveably inserted in this half shell (3, 4) in the direction toward the at least one powder receiving depression (9), wherein the plunger (10) is formed as a pricker for opening an active ingredient depression (101) of a blister element (100) inserted into the powder inhaler (1); and
B. on one of the half shells (3, 4) one of the sidewall sections (32, 42) at the level of the powder receiving depression (9) comprises a cutout (32') whose width and height correspond to a width and height of a blister element (100) determined for insertion into the powder inhaler (1), wherein
on the sidewall section (32, 42) that, in a joined arrangement of the half shells (3, 4), is positioned above the cutout (32'), the second half shell (3, 4) that does not have the cutout (32') comprises a projection (32") that is designed to close off the cutout (32') when a blister element (100) is received in the powder inhaler (1).

2. Powder inhaler (1) according to claim 1,
**characterized in that**
the powder inhaler (1) is an injection molded part.

3. Powder inhaler (1) at least according to claim 1 or 2,
**characterized in that**
the powder inhaler (1) comprises at least two powder receiving depressions (9), wherein at least one air inlet channel extends to each one of the powder receiving depressions (9), respectively.

4. Powder inhaler (1) according to one of the claims 1 to 3,
**characterized in that**
the at least one air swirling structure is formed by several vanes (5) projecting from the guide webs (6) into the respective air supply channel.

5. Powder inhaler (1) according to claim 4,
**characterized in that**
the vanes (5) are vanes (5) that are alternatingly arranged on two oppositely positioned and/or side by side neighboring guide webs (6) and form obstacles for an air stream that is flowing along the fluid path.

6. Powder inhaler (1) according to one of the claims 1 to 5,
**characterized in that**
the outlet is formed as a mouthpiece or a nose piece.

7. Powder inhaler (1) according to claim 6,
**characterized in that**
the mouthpiece is formed as a spout (20) or the nose piece is formed as a curved nose tube (21).

8. Powder inhaler (1) according to one of the claims 1 to 7,
**characterized in that**
at least one of the de-agglomeration structures (17) in the outlet area of the two half shells (3, 4) is formed by webs (17) that extend from oppositely positioned wall sections (22) into the fluid path.

9. Powder inhaler (1) according to claim 8,
**characterized in that**
the webs (17) projecting into the fluid path are alternatingly arranged on the two wall sections (22).

10. Powder inhaler (1) according to at least one of the claims 1 to 9, **characterized in that**
at least one of the de-agglomeration structures (17') in the outlet area adjoins the powder deposition and release area and is formed by at least one deflection web (17') which is arranged in one of the half shells (3, 4).

11. Powder inhaler (1) according to claim 10,
**characterized in that**
the at least one deflection web (17') is arranged in the half shell (3, 4) that comprises the powder receiving depression (9).

12. Powder inhaler (1) according to one of the preceding claims,
**characterized in that**
the half shell (3, 4) that comprises the powder receiving depression (9) comprises at least one stop (12) that is provided for framing the blister element (100) predetermined for reception in the powder inhaler (1).

13. Powder inhaler (1) according to one of the preceding claims,
**characterized in that**
both half shells (3, 4) have a ribbing (18) at the sidewall sections (32, 42) at the level of the powder receiving depression (9) opposite the cutout (32') and the projection (32").

14. Powder inhaler (1) according to one of the preceding claims,
**characterized in that**
the half shell (3, 4) which comprises the powder receiving depression (9) has a projection that is designed to engage a corresponding cutout (103) of the blister element (100) predetermined for reception in the powder inhaler (1).

15. Powder inhaler (1) according to claim 14,
**characterized in that**
at least one additional half shell (15) is connected by means of at least one hinge (16) to one of the half shells (3, 4).

16. Powder inhaler (1) according to one of the preceding claims,
**characterized in that**
at least one additional half shell (15) is connected by means of at least one hinge (16) to one of the half shells (3, 4).

17. Powder inhaler (1) according to claim 16,
**characterized in that**
the at least one additional half shell (15) is connected to the half shell (3, 4) that does not comprise the at least one air inlet (8).

18. Powder inhaler (1) according to claim 16 or 17,
**characterized in that**
the additional half shell (15) comprises a base which corresponds to the base of the further half shells (3, 4) and which has a size for storage of at least one blister element (100) predetermined for reception in the powder inhaler (1).

19. Powder inhaler (1) according to one of the preceding claims,
**characterized in that**
on the half shells (3, 4, 15) locking means for joining at least the first half shell (3) with the second half shell (4) are provided.

20. Powder inhaler (1) according to one of the claims 16 to 19,
**characterized in that**
on the at least one additional half shell (15) locking means for joining with one of the half shells (3, 4, 15) are provided.

21. Powder inhalation set (1, 100)
**characterized in that**
it comprises a powder inhaler (1) according to at least one of the claims 1 to 20 and a blister element (100) dimensioned for reception in the powder inhaler (1), wherein the blister element (100) comprises a support film with at least one depression (101) that is formed for receiving at least one active ingredient powder that is inhalable, and a cover plate that closes off the depression (101), wherein the at least one depression (101) of the blister element (100) in size and position is formed correspondingly for reception in the at least one powder receiving depression (9) of the powder inhaler (1).

22. Powder inhalation set (1, 100) according to claim 21,
**characterized in that**
for opening the depression (101), the cover plate is designed to be removable from the support plate through a cutout (32') in a sidewall section (32, 42) at the level of the powder receiving depression (9) of the powder inhaler (1),
and/or
for opening the depression (101), the cover plate is penetrable by a plunger (10), which is arranged in the half shell (3, 4) of the powder inhaler (1) opposite the powder receiving depression (9), is elastically moveable, and designed as a pricker.

## Revendications

1. Inhalateur de poudre (1), comprenant
- deux demi-coques (3, 4) qui sont reliées par au moins un film-charnière (16) et formées en une pièce, les deux demi-coques (3, 4) dans une disposition assemblée entourant une zone d'entrée d'air, une zone de dépôt et de libération de poudre et une zone de sortie, par laquelle passe un trajet fluidique, **moyennant quoi**
- au moins l'une des demi-coques (3, 4) présente, dans la zone d'entrée d'air, au moins une entrée d'air (8),
- dans la zone de dépôt et de libération de poudre, une des demi-coques (3, 4) présente au moins un creux récepteur de poudre (9), dans lequel un creux (101) d'un élément blister (100) dimensionné pour entrer dans l'inhalateur de poudre (1) est amovible, lequel présente un film support présentant au moins le creux (101) qui est formé pour recevoir au moins un principe actif en poudre inhalable, un couvercle de l'élément blister (100) obturant le creux (101) qui est formé en conséquence, en termes de taille et de position, pour s'insérer dans l'au moins un creux récepteur de poudre (9) de l'inhalateur de poudre (1),
- la zone de sortie présente au moins une structure de désagglomération (17, 17') et une sortie pour l'aérosol qui est formée par les demi-coques (3, 4),
- les deux demi-coques (3, 4) sont plates et présentent une base oblongue essentiellement rectangulaire, en forme de trapèze, en forme de goutte ou ovale, qui est délimitée par une paroi ou des sections de paroi,
- l'au moins une entrée d'air (8) est disposée sur l'une des sections de paroi (31, 41), écartée de la sortie,
- au moins deux, de préférence plusieurs entretoises directrices (6) se trouvent sur au moins l'une des deux demi-coques (3, 4), lesquelles s'étendent de la section de paroi (31, 41) écartée de la sortie et/ou des sections de paroi latérale (32, 42) adjacentes à la section de paroi (31, 41) écartée de la sortie, jusqu'à l'au moins un creux récepteur de poudre (9), les entretoises directrices (6) de l'au moins une demi-coque (3, 4) s'étendant dans leur hauteur jusqu'à l'autre demi-coque (3, 4) ou jusqu'aux entretoises directrices (6) de l'autre demi-coque (3, 4), deux entretoises directrices (6) adjacentes formant un conduit d'amenée d'air et limitant le trajet fluidique dans la zone d'entrée d'air,
- les entretoises directrices (6) adjacentes sont disposées en forme d'éventail,
- chaque conduit d'amenée d'air présente une section se resserrant vers l'au moins un creux récepteur de poudre (9),
- l'au moins un creux récepteur de poudre (9) est délimité, le long du trajet fluidique, des deux côtés par des parois de guidage (7) qui s'étendent des entretoises directrices (6) les plus extérieures vers celles menant vers l'au moins un creux de poudre (9),
- au moins une structure de tourbillonnement d'air est disposée dans chaque conduit d'amenée d'air, l'inhalateur de poudre (1) présentant au moins l'une des caractéristiques A et B suivantes :
A. Dans la zone de dépôt et de libération de poudre, la demi-coque (3, 4) ne présentant pas l'au moins un creux récepteur de poudre (9), présente, sur une zone opposée à l'au moins un creux récepteur de poudre (9), un poinçon (10) qui est utilisé de façon mobile au moyen d'un embout élastique (14) dans cette demi-coque (3, 4) en direction de l'au moins un creux récepteur de poudre (9), le poinçon (10) étant formé comme un dôme pour l'ouverture d'un creux de principe actif (101) d'un élément blister (100) inséré dans l'inhalateur de poudre (1) et
B. sur une des demi-coques (3, 4), une des sections de paroi latérale (32, 42) présente, au niveau du creux récepteur de poudre (9), une encoche (32'), dont la largeur et la hauteur correspondent à une largeur et une hauteur d'un élément blister (100) prédéfini pour être inséré dans l'inhalateur de poudre (1), la deuxième demi-coque (3, 4) qui ne présente pas l'encoche (32'), sur la section de paroi latérale (32, 42) qui repose au-dessus de l'encoche (32') dans une disposition assemblée des demi-coques (3, 4), présentant une saillie (32") qui est formée pour obturer l'encoche (32') lorsqu'un élément blister (100) est inséré dans l'inhalateur de poudre (1).

2. Inhalateur de poudre (1) conformément à la revendication n°1,
**caractérisé par le fait que**
l'inhalateur de poudre (1) est une pièce moulée par injection.

3. Inhalateur de poudre (1) conformément au moins à la revendication n°1 ou n°2,
**caractérisé par le fait que**
l'inhalateur de poudre (1) présente au moins deux creux récepteurs de poudre (9), au moins un conduit d'amenée d'air menant vers chacun des creux récepteurs de poudre (9).

4. Inhalateur de poudre (1) conformément à l'une des revendications n°1 à n°3,
**caractérisé par le fait que**
au moins une structure de tourbillonnement d'air est formée de plusieurs pales (5) en saillie par rapport aux entretoises directrices (6) dans le conduit d'amenée d'air respectif.

5. Inhalateur de poudre (1) conformément à la revendication n°4,
**caractérisé par le fait que**
les pales (5) sont des pales (5) disposées en alternance sur deux entretoises directrices (6) opposées et/ou adjacentes et constituent des chicanes pour un flux d'air circulant le long du trajet fluidique.

6. Inhalateur de poudre (1) conformément à l'une des revendications n°1 à n°5,
**caractérisé par le fait que**
la sortie est constituée sous forme d'embout ou de clavette.

7. Inhalateur de poudre (1) conformément à la revendication n°6,
**caractérisé par le fait que**
l'embout est réalisé sous forme de bec (20) ou que la clavette est réalisée sous forme d'embout tubulaire (21) coudé.

8. Inhalateur de poudre (1) conformément à l'une des revendications n°1 à n°7,
**caractérisé par le fait que**
au moins l'une des structures de désagglomération (17) est formée, dans la zone de sortie des demi-coques (3, 4) par des entretoises (17) en saillie dans le trajet fluidique sur les sections de paroi latérale (22) opposées.

9. Inhalateur de poudre (1) conformément à la revendication n°8,
**caractérisé par le fait que**
les entretoises (17) en saillie dans le trajet fluidique sont disposées en alternance sur les deux sections de paroi (22).

10. Inhalateur de poudre (1) conformément au moins à l'une des revendications n°1 à n°9,
**caractérisé par le fait que**
au moins l'une des structures de désagglomération (17') est adjacente, dans la zone de sortie, à la zone de dépôt et de libération de poudre et formée par au moins une entretoise de renvoi (17') qui est disposée dans l'une des demi-coques (3, 4).

11. Inhalateur de poudre (1) conformément à la revendication n°10,
**caractérisé par le fait que**
l'au moins une entretoise de renvoi (17') est disposée dans la demi-coque (3, 4) qui présente le creux récepteur de poudre (9).

12. Inhalateur de poudre (1) conformément à l'une des revendications précédentes,
**caractérisé par le fait que**
la demi-coque (3, 4) qui présente le creux récepteur de poudre (9), présente au moins une butée (12) qui est disposée pour délimiter l'élément blister (100) prédéfini pour être inséré dans l'inhalateur de poudre (1).

13. Inhalateur de poudre (1) conformément à l'une des revendications précédentes,
**caractérisé par le fait que**
les deux demi-coques (3, 4) aux sections de paroi latérale (32, 42) présentent, au niveau du creux récepteur de poudre (9), une rainure (18) par rapport à l'encoche (32') et à la saillie (32").

14. Inhalateur de poudre (1) conformément à l'une des revendications précédentes,
**caractérisé par le fait que**
la demi-coque (3, 4) qui présente le creux récepteur de poudre (9), présente une saillie qui est formée pour s'insérer avec une cavité correspondante (103) de l'élément blister (100) prédéfini pour être inséré dans l'inhalateur de poudre (1).

15. Inhalateur de poudre (1) conformément à la revendication n°14,
**caractérisé par le fait que**
la saillie est une saillie en forme de coupole (18').

16. Inhalateur de poudre (1) conformément à l'une des revendications précédentes,
**caractérisé par le fait que**
au moins une autre demi-coque (15) est coupée sur l'une des demi-coques (3, 4) au moins par une charnière (16).

17. Inhalateur de poudre (1) conformément à la revendication n°16,
**caractérisé par le fait que**
l'au moins une autre demi-coque (15) est coupée sur la demi-coque (3, 4) sans entrées d'air (8).

18. Inhalateur de poudre (1) conformément à la revendication n°16 ou n°17,
**caractérisé par le fait que**
l'autre demi-coque (15) présente une base qui correspond à la base des autres demi-coques (3, 4) et qui présente une taille permettant le stockage d'au moins un élément blister (100) prédéfini pour être inséré dans l'inhalateur de poudre (1).

19. Inhalateur de poudre (1) conformément à l'une des revendications précédentes,
**caractérisé par le fait que**
des moyens d'encliquetage pour l'assemblage d'au moins la première demi-coque (3) avec la deuxième demi-coque (4) sont prévus sur les demi-coques (3, 4, 15).

20. Inhalateur de poudre (1) conformément à l'une des revendications n°16 à n°19,
**caractérisé par le fait que**
des moyens d'encliquetage pour l'assemblage avec l'une des demi-coques (3, 4, 15) sont prévus sur l'au moins une autre demi-coque (15).

21. Set d'inhalation de poudre (1, 100)
**caractérisé par le fait que**
il présente un inhalateur de poudre (1) conformément au moins à l'une des revendications n°1 à n°20 et un élément blister (100) dimensionné pour être inséré dans l'inhalateur de poudre (1), l'élément blister (100) présentant un film support avec au moins un creux (101) qui est formé pour recevoir au moins un principe actif en poudre inhalable et un couvercle obturant le creux (101), l'au moins un creux (101) de l'élément blister (100) étant formé en conséquence, en termes de taille et de position, pour s'insérer dans l'au moins un creux récepteur de poudre (9) de l'inhalateur de poudre (1).

22. Set d'inhalation de poudre (1, 100) conformément à la revendication n°21,
**caractérisé par le fait que**
le couvercle pour l'ouverture du creux (101) depuis la plaque support est formé de façon amovible par une encoche (32') dans une section de paroi latérale (32, 42) au niveau du creux récepteur de poudre (9) de l'inhalateur de poudre (1),
et/ou
le couvercle pour l'ouverture du creux (101) est pénétrable par un poinçon (10) disposé à l'opposé du creux récepteur de poudre (9), élastiquement déplaçable et réalisé sous forme de mandrin (10).
